**Europäisches Patentamt**

**European Patent Office**

**Office européen des brevets**

(19)

(11) Veröffentlichungsnummer: **0 074 610**

**B1**

(12) **EUROPÄISCHE PATENTSCHRIFT**

(45) Veröffentlichungstag der Patentschrift:
20.05.87

(51) Int. Cl.⁴: **G 01 N 33/92**

(21) Anmeldenummer: **82108259.1**

(22) Anmeldetag: **08.09.82**

(54) **Verfahren zur selektiven extrakorporalen Präzipitation von Low Density Lipoproteinen aus Vollserum oder Plasma.**

(30) Priorität: 10.09.81 DE 3135814
13.05.82 DE 3217925

(43) Veröffentlichungstag der Anmeldung:
23.03.83 Patentblatt 83/12

(45) Bekanntmachung des Hinweises auf die Patenterteilung:
20.05.87 Patentblatt 87/21

(84) Benannte Vertragsstaaten:
AT BE CH DE FR GB IT LI LU NL SE

(56) Entgegenhaltungen:
EP-A-0 035 211
EP-A-0 044 694
DE-A-2 027 087
DE-B-2 345 994
US-A-4 103 685
US-A-4 215 993
US-A-4 223 672

JOURNAL OF LIPID RESEARCH, Band 11, 1970 M.
BURSTEIN et al.: "Rapid method for the isolation of
lipoproteins from human serum by precipitation
with polyanions", Seiten 583-595
CLINICAL CHEMISTRY, Band 26, Nr. 13, 1980
EASTON P.N.M. DEMACKER et al.: "Measurement
of high-density lipoprotein cholesterol in serum:
comparison of six isolation methods combined
with enzymic cholesterol analysis", Seiten 1780-1786

(73) Patentinhaber: Intermedicat GmbH,
Gerliswilstrasse 45, CH- 6020 Emmenbrücke (CH)

(72) Erfinder: Seidel, Dietrich, Prof., Dahlmannstrasse
23, D-3400 Göttingen (DE)
Erfinder: Wieland, Heinrich, Dr., Wiesenweg 2,
D-3401 Waake (DE)
Erfinder: Rath, Dieter, Franz- Gleim- Strasse 67,
D-3508 Melsungen (DE)
Erfinder: Rosskopf, Gerhard, Dr.,
Heiligenbergstrasse 29, D-3501 Fuldabrück-
Dörnhagen (DE)

(74) Vertreter: von Kreisler, Alek, Dipl.- Chem.,
Deichmannhaus am Hauptbahnhof, D-5000 Köln 1
(DE)

(56) Entgegenhaltungen: (Fortsetzung)
PATENT ABSTRACTS OF JAPAN, Band 5, Nr. 89, 10.
Juni 1981; & JP - A- 56 33017

## Beschreibung

Die Fortschritte auf dem Gebiet der Analytik des Lipoproteinsystems der letzten Jahre haben ergeben, daß die in älteren epidemiologischen Daten erhobenen engen Korrelationen zwischen Plasmacholesterinkonzentrationen und dem Risiko einer frühzeitigen Atherosklerose, speziell der Koronarsklerose, im wesentlichen durch cholesterinreiche β-Lipoproteine gegeben sind. Im Blut des Menschen finden sich normalerweise ca. 70 - 80% des gesamten Cholesterins an die low density bzw. β-Lipoproteine gebunden, der Rest verteilt sich auf andere Lipoproteinfraktionen (im wesentlichen VLDL, HDL und Chylomikronen) (LDL = = Low Density Lipoproteine; VLDL = Very Low Density Lipoproteine; HDL = High Density Lipoproteine). In Krankheitsprozessen, die mit einem gestörten Fettstoffwechsel bzw. erhöhten Plasmalipidkonzentrationen einhergehen und die zur frühzeitigen Atherosklerose führen, kann sich der prozentuale Anteil des LDL- oder β-Cholesterins (LDL = β-Lipoprotein) am Gesamtcholesterin sogar noch weit erhöhen. Das heisst, eine Hypercholesterinämie ist in der Regel durch eine Hyper-β-Lipoproteinämie hervorgerufen. In der Vergangenheit hat es daher auch nicht an Versuchen gefehlt

1. die low density Lipoproteine selektiv zu messen und

2. solche, möglichst selektiv aus der Zirkulation zu eliminieren.

Sowohl das eine als auch das andere ist bisher unter Berüksichtigung des Standes der Technik nicht befriedigend gelöst worden. Die üblichen Verfahren, low density Lipoproteine zu quantifizieren beruhen entweder auf der Verwendung der Trennung des Lipoproteinspektrums in Dichteklassen unter Zuhilfenahme der Ultrazentrifuge oder auf der Trennung des Lipoproteinspektrums im elektrischen Feld, ein Verfahren, das man sich bei der sog. Lipoproteinelektrophorese zunutze macht. Weiterhin auf Präzipitationsverfahren, die darauf basieren, daß apo-B-haltige Lipoproteine (VLDL und LD) durch Polyanicnen und divalente Kationen von nicht-apo-B-haltigen Lipoproteinen durch Ausfällung der erstgenannten getrennt bestimmt werden können. Bei dem Apo-B-Protein handelt es sich um den hauptsächlichen Proteinanteil der low density bzw. β-Lipoproteine als auch der VLDL bzw. prä-β-Lipoproteine und Chylomikronen. Das letztgenannte Vefahren (Präzipitationstechniken) war bisher nicht geeignet, VLDL von LDL zu trennen. Die beiden erstgenannten Verfahren Ultrazentrifugation Havel, R.J., Eder H.A. und Bragdon J.H.: The Distribution and Chemical Composition of Ultracentrifugally Separated Lipoproteins in Human Serum, J.Clin.Invest. 34, 1345, 1955 und Elektrophorese Wieland H. und Seidel D.: Fortschritte in der Analytik des Lipoproteinmusters, Inn.Med. 5, 290 - 300,1978.. hatten bei der Anwendung größerer Routineserien den Nachteil, daß sie entweder zu kostspielig und zeitaufwendig oder nicht automatisierbar waren. Zusätzlich war eine direkte Messung des low density Cholesterins nur nach Isolierung der Fraktionen, üblicherweise unter Verwendung der Ultrazentrifuge, möglich. Die rechnerische Ermittlung des low density Cholesterinanteils über die Elektrophorese setzt gewisse Prämissen der Proteinlipidzusammensetzung voraus. Gleiches gilt für die Verfahren, die sich die Präzipitationstechniken zunutze machen. Die Präzipitationstechniken der üblichen Weise haben zusätzlich den Nachteil, daß sie das Lipoproteinspektrum nur in zwei Hauptfraktionen (apo-B-haltige und nicht-apo-B-haltige) auftrennen, also keine Unterscheidung oder Trennung von VLDL und LDL möglich ist.

Die in der US-PS4 215993 beschriebene Verfahrensweise, Lipoproteine allgemein in ihrem isoelektrischen Punkt zu fällen, bezieht sich im Unterschied zu dem vorliegenden erfindungsgemäßen Verfahren auf die Verwendung von Natriumphosphorwolframat. Dieses Polyanion ist unphysiologisch. Darüber hinaus gelingt es unter Verwendung von Phosphorwolframat nicht, low density Lipoproteine selektiv zu eliminieren oder zu bestimmen, also die Hauptzielsetzung der vorliegenden Erfindung wird nicht erreicht. Daß dieses nicht gelingt, wurde in der US-PS 4 125 993 u.a. dadurch aufgezeigt, daß man empfiehlt, die low density Lipoproteine nach einer Rechenformel (Friedewald-Formel) aus dem sog. HDL-Cholesterin unter zusätzlicher Berücksichtigung des Gesamtcholesterins und der Triglyceride zu errechnen. Im wesentlichen unterscheidet sich die isoelektrische Punktfällung unter Verwendung von Phosphorwolframsäure, wie in der US-PS 4 125 993 angegeben, in ihrem Resultat nicht von der üblichen Polyanionpräzipitation mit Verwendung zweiwertiger Kationen. Eine komplette Fällung von VLDL und LDL in dem genannten Verfahren wird nur erreicht, wenn Polymere, wie z.B. Polyvinylpyrolidon oder Polyäthylenglykol genau definierten Molekulargewichts der Präzipitationslösung zugesetzt werden. Daß dies in einem extrakorporalen System nicht zu verwirklichen ist, steht außer Frage.

Im übrigen ist darauf hinzuweisen, daß in der US-PS 4 125 993 irreführenderweise die Gesamtheit von β-Lipoproteinen, prä-β-Lipoproteinen und Chylomikronen als low density Lipoproteine bezeichnet wird. Da erklärtes Ziel der US-PS 4 125 993 ist, high density Lipoproteine im Serum zu bestimmen, muß Wert darauf gelegt werden, daß die neben high density Lipoproteinen noch vorkommenden VLDL, Chylomikronen und LDL durch dieses Präzipitationsverfahren eliminiert werden. Eine spezifische Präzipitation der wirklichen low density Lipoproteine oder β-Lipoproteine wurde weder beabsichtigt noch erreicht.

Die vorliegende Erfindung stellt sich daher die Aufgabe, ein Verfahren und eine Vorrichtung zu entwickeln, die es gestatten, selektiv und mit hoher Kapazität low density Lipoproteine oder β-Lipoproteine aus dem Blut, d.h. dem Vollserum oder Bestandteilen des Blutes wie Plasma zu entfernen und dieses Prinzip sowohl für nicht therapeutische als auch diagnostische Zwecke zu verwenden.

Gelöst wird die Aufgabe durch die Zurverfügungstellung eines dafür geeigneten Verfahrens.

Die Erfindung betrifft ein Verfahren zur spezifischen extrakorporalen Präzipitation von low density-Lipoproteinen oder β-Lipoproteinen aus Vollserum oder Plasma für nicht therapeutische Zwecke, das dadurch gekennzeichnet ist, daß zu dem Vollserum oder Plasma Heparin in einem Puffer gegeben wird und der gebildete β-Lipoprotein-Heparin-Komplex im isoelektrischen Punkt bei einem pH-Wert von 5,05 bis 5,25 ausgefällt und anschließend abgetrennt oder das Präzipit bzw. das Filtrat zu diagnostischen Zwecken weiter analysiert wird.

Das Verfahren beruht auf der absolut spezifischen Ausfällung der β-Lipoproteine nach Vernetzung mit Heparin durch Ausfällung im isoelektrischen Punkt des Komplexes bei einem pH-Wert von 5,05 bis 5,25, insbesondere bei einen pH-Wert von 5,13. Neben der Spezifität zeichnet sich dieses Verfahren weiterhin dadurch aus, daß als einzige Substanz die zur Präzipitation der low density Lipoproteine verwendet wird, eine im Körper selbst produzierte Substanz (Heparin) eingesetzt wird. Fällung im isoelektrischen Punkt durch Senkung des pH unter Verwendung von Puffern kann durch geeignete Filtration und pH-Angleichung nach der isoelektrischen Punktfällung leicht auf physiologische Verhältnisse hin korrigiert werden.

Das Heparin wird in einem Puffer gelöst und mit dem Vollserum oder Plasma vermischt. Der Puffer dient zur Einstellung und Stabilisierung der angegebenen pH-Werte. Alle Puffergemische, die eine Stabilität in dem angegebenen pH-Bereich sichern, sind prinzipiell für die Anwendung des Verfahrens brauchbar.

Das Verhältnis von Puffer zu Serum bzw. Plasma bzw. Blutvolumen kann zwischen 1 : 5 bis 5 : 1 liegen. Als günstigste Volumenverhältnisse für die selektive Elimination der low density Lipoproteine aus dem Blut für diagnostische Zwecke hat sich ein Verhältnis zwischen 2 bis 10: 1 Puffer zu Serum ergeben.

Die Heparinkonzentration ist kein besonders kritischer Punkt. Günstigerweise wird man jedoch auf Heparinkonzentrationen zurückgreifen, die nahezu komplett durch die β-Lipoproteine mit auspräzipitiert werden. Beispielsweise sollte eine gegebene Serummenge im günstigsten Falle mit ca 10 Vol-% einer Heparin-Puffer-Lösung, die 5OO-5000 E/ml enthält, versetzt werden.

Beim Zusammenbringen von z.B. einer Mischung von gleichen Teilen des Vollserums oder Plasmas mit einem Phosphat-Citrat-Puffer, in dem das Heparin gelöst ist, bei dem sich einstellenden pH-Wert von etwa 5,13, erfolgt die Ausfällung sofort und vollständig und ist spezifisch.

Das bedeutet, daß nur die β-Lipoproteine oder low density Lipoproteine ausgefällt werden. Die ausgefällten β-Lipoproteine können beispielsweise durch einen Membranfilter der Porengröße von < 2 μm, vorzugsweise 0,4 - 0,8 μm, bzw. durch einfache Zentrifugation in einem Continuous-Flow-Verfahren vom verbleibenden Serum abgetrennt werden.

Bei der Diagnostik kann sowohl der von β-Lipoproteinen befreite Serumrückstand, als auch der ausgefällte Komplex von β-Lipoproteinen und Heparin weiteren Untersuchungen unterzogen werden.

Die Spezifität des erfindungsgemäßen Verfahrens wurde sowohl mit quantitativen immunologischen Techniken sowie der Ultrazentrifugation als auch quantitativen Lipoproteinelektrophorese überprüft. Die hier beschriebene spezifische pH-Fällung von low density Lipoproteinen führt zu einer kompletten Präzipitation dieser β-Lipoproteinklasse. Im verbleibenden Filtrat finden sich lediglich HDL und VLDL an Lipoproteinen.

Die Spezifität des erfindungsgemäßen Verfahrens und die Anwendung ausschließlich physiologischer Substanzen erlaubt die selektive Elimination von LDL aus dem Plasma in vitro und eine Bestimmung dieser Fraktion entweder aus Differenzberechnung zum Gesamtcholesterin und dem verbleibenden Filtrat oder durch die direkte Cholesterinbestimmung am Präzipität bzw. durch Trübungsmessung des entstehenden Präzipitats. Diese direkte Bestimmung des LDL- bzw. β-Cholesterins ist neu und bisher in keinem konkurrierenden Verfahren beschrieben.

Das erfindungsgemäße Verfahren wurde sowohl mit der präparativen Ultrazentrifuge als auch mit der quantitativen Lipoproteinelektrophorese verglichen und hat mit beiden Verfahren Korrelationskoeffizienten von über 0.98 in einer Serie von über 100 Patientenseren ergeben. Die Spezifität des erfindungsgemäßen Verfahrens ist der Ultrazentrifuge deutlich überlegen. Gegenüber der Elektrophorese hat das erfindungsgemäßen Verfahren den Vorteil der Möglichkeit einer direkten Cholesterinbestimmung der β-Lipoproteine. Das erfindungsgemäße Verfahren läßt sich leicht sowohl in einem Diskretautomaten als auch in einem Continuous Flow-Automaten als mechanisierte Methode betrieben. Die Präzision in der Serie wie auch von Tag zu Tag liegt unter 2 %.

Die Erfindung wird weiter in den folgenden Ausführungsbeispielen erläutert.

1. Plasma wird mit einer gleichen Menge eines Citrat-, Phosphat- oder Acetatpuffers (0.05 M pH 4,15) gemischt. Nach Hinzufügen einer Heparinmenge (5000 E/ml), die 1/10 der eingesetzten Plasmamenge beträgt, bildet sich ein gelbweißer Niederschlag, der aus den ausgefällten β-Lipoproteinen und hauptsächlich Fibrinogen besteht.

2. 100 µl Serum werden mit 1 ml einer Lösung gemischt, die folgendermaßen zusammengesetzt ist: 0,0641 M Na-Citrat, pH 5,04 1%ig an Liqemin-25 000 (Roche). Das Gemisch wird für 10 Minuten in einer Eppendorf-Zentrifuge zentrifugiert. Der Chlolesteringehalt des Überstandes entspricht dem Cholesteringehalt von VLDL und HDL. Die LDL sind ausgefällt worden und lassen sich aus der Differenz Vollserumcholesterin-VLDL + HDL-Cholesterin errechnen.

**Patentansprüche**

1. Verfahren zur selektiven extrakorporalen Präzipitation von low density Lipoproteinen oder β-Lipoproteinen aus Vollserum oder Plasma für nicht therapeutische Zwecke, dadurch gekennzeichnet, daß zu dem Vollserum oder Plasma Heparin in einem Puffer gegeben wird und der gebildete β-Lipoprotein-Heparin-Komplex im isoelektrischen Punkt bei einem pH-Wert von 5,05 bis 5,25 ausgefällt und anschließend abgetrennt oder das Präzipität bzw. das Filtrat zu diagnostischen Zwecken weiter analysiert wird.

2. Verfahren nach Anspruch 1, dadurch gekennzeichnet, daß die Ausfällung des β-Lipoprotein-Heparin-Komplexes bei einem pH-Wert von 5,13 erfolgt.

3. Verfahren nach Ansprüchen 1 und 2, dadurch gekennzeichnet, daß als Puffer Phosphatpuffer, Citratpuffer, Lactatpuffer, Acetatpuffer oder deren Gemisch verwendet werden.

4. Verfahren nach Ansprüchen 1 - 3, dadurch gekennzeichnet, daß das Verhältnis von Puffer zu Serum oder Puffer zu Plasma oder Puffer zu Blutvolumen zwischen 1 : 5 bis 5 : 1 liegt.

5. Verfahren nach Ansprüchen 1 - 3, dadurch gekennzeichnet, daß das Volumenverhältnis zwischen Puffer und Serum für diagnostische Zwecke zwischen 2 bis 10 :1 liegt.

6. Verfahren nach Ansprüchen 1 - 5, dadurch gekennzeichnet, daß die Heparinkonzentration der Konzentration an β-Lipoproteinen entspricht.

7. Verfahren nach Ansprüchen 1 - 6, dadurch gekennzeichnet, daß nach dem Abtrennen des ausgefällten β-Lipoprotein-Heparin-Komplexes das verbleibende Serum oder Plasma mit normalem Blut vermischt wird.

8. Verfahren nach Ansprüchen 1 - 6, dadurch gekennzeichnet, daß nach dem Abtrennen des ausgefällten β-Lipoprotein-Heparin-Komplexes das verbleibende Filtrat zu diagnostischen Zwecken weiter analysiert wird oder das Präzipitat bzw. Teile dessen quantitativ bestimmt werden.

**Claims**

1. A process for the selective extracorporeal precipitation of low density lipoproteins from full serum or plasma for non-therapeutical purposes, characterized in that heparin in a buffer is added to the full serum or plasma and the β-lipoproteinheparin complex formed is precipitated at the isoelectric point at a pH value of from 5.05 to 5.25 and subseguently separated or the precipitate and/or filtrate is/are further analyzed for diagnostic purposes.

2. The process according to claim 1, characterized in that the precipitation of the β-lipoproteinheparin complex is effected at a pH value of 5.13.

3. The process according to claims 1 and 2, characterized in that phosphate buffers, citrate buffers, lactate buffers, acetate buffers or a mixture thereof are used as the buffer.

4. The process according to claims 1 to 3, characterized in that the ratio of buffer to serum or buffer to plasma or buffer to blood volume is between 1:5 to 5:1.

5. The process according to claims 1 to 3, characterized in that the ratio by volume between buffer and serum for diagnostic purposes is between 2 and 10 :1.

6. The process according to claims 1 to 5, characterized in that the heparin concentration corresponds to the concentration of β-lipoproteins.

7. The process according to claims 1 to 6, characterized in that after the separation of the precipitated β-lipoprotein-heparin complex the remaining serum or plasma is admixed with normal blood.

8. The process according to claims 1 to 6, characterized in that after the separation of the precipitated β-lipoprotein-heparin complex the remaining filtrate is further analyzed for diagnostical purposes or the precipitate or parts thereof are quantitatively determined.

**Revendications**

1. Procédé pour la précipitation sélective extracorporelle de lipoprotéines ou de bêta-lipoprotéines basse densité du sérum ou du plasma sanguin, à des fins non médicinales, caractérisé en ce que l'on ajoute au sérum ou au plasma sanguin de l'héparine dans un tampon, que l'on précipite au point isoélectrique à un pH allant de 5,05 à 5,25 le complexe formé de bêta-lipoprotéine/héparine puis que l'on sépare ou bien que l'on soumet à des analyses complémentaires le filtrat à des fins diagnostiques.

2. Procédé selon la revendication 1, caractérisé en ce que la précipitation du complexe de bêta-lipoprotéine/ héparine a lieu à un pH de 5,13.

3. Procédé selon les revendications 1 et 2, caractérisé en ce qu'on utilise comme tampon du tampon de phosphate, du tampon de citrate, du tampon lactate, du tampon d'acétate ou leur mélange.

4. Procédé selon les revendications 1 à 3, caractérisé en ce que le rapport du tampon au sérum ou du tampon au plasma ou du tampon au volum de sang se situe entre 1:5 et 5:1.

5. Procédé selon les revendications 1 à 3, caractérisé en ce que le rapport en volume entre le tampon et le sérum se situe à des fins diagnostiques entre 2 et 10:1.

6. Procédé selon les revendications 1 à 5, caractérisé en ce que la concentration de l'héparine correspond à la concentration en bêta-lipoprotéines.

7. Procédé selon les revendications 1 à 6, caractérisé en ce que, après la séparation du complexe précipité de bêta-lipoprotéine/héparine, on mélange avec du sang normal le sérum ou le plasma restant.

8. Procédé selon les revendications 1 à 6, caractérisé en ce que, après la séparation du complexe précipité de bêta-lipoprotéine/héparine, on soumet le filtrat restant à des analyses supplémentaires à des fins diagnostiques ou bien on effectue des déterminations quantitatives du précipité ou de parties de celui-ci.